# EUROPÄISCHE PATENTSCHRIFT

(11) **EP 2 735 322 B1**
(45) Veröffentlichungstag und Bekanntmachung des Hinweises auf die Patenterteilung: **27.05.2015**
(21) Anmeldenummer: 13194266.6
(22) Anmeldetag: 25.11.2013
(51) Int. Cl.: A61M 1/16

(54) **Kartuschenhalterung einer Dialysemaschine mit integrierter Positionierhilfe**
Cartridge holder for a dialysis machine with integrated positioning aid
Support de cartouche d'une machine de dialyse avec aide au positionnement intégrée

(30) Priorität: 26.11.2012 DE 102012111429
(43) Veröffentlichungstag der Anmeldung: 28.05.2014
(73) Patentinhaber: B. Braun Avitum AG, 34212 Melsungen (DE)
(72) Erfinder: Iske, Andreas, 34320 Söhrewald (DE)
(74) Vertreter: Winter, Brandl, Fürniss, Hübner, Röss, Kaiser, Polte - Partnerschaft mbB

(56) Entgegenhaltungen:
- EP-A2- 1 002 550
- WO-A1-01/64312
- WO-A1-99/37342
- WO-A1-2010/121821
- DE-A1- 10 059 966
- DE-C1- 19 925 297

## Beschreibung

Die vorliegende Erfindung betrifft einen Kartuschenhalter einer Dialysemaschine und insbesondere eine an einer Dialysemaschine angeordnete / integrierte Halterung oder Halteeinrichtung für eine Bikarbonatkartusche mit einer Kartuschen - Positionierhilfe gemäß dem Oberbegriff des Anspruchs 1. Des Weiteren betrifft die vorliegende Erfindung eine Dialysemaschine mit einer solchen Halterung / Halteeinrichtung.

### Hintergrund der Erfindung

In Dialysemaschinen wird eine Dialysierflüssigkeit aufbereitet, die durch einen Dialysator geleitet wird, um Blut eines Dialysepatienten Schadstoffe (insbesondere urämische Toxine) zu entziehen. Hierfür wird das Dialysegerät in der Regel unmittelbar vor einer jeweiligen Behandlung desinfiziert, um ein Infektionsrisiko auf Seiten des Patienten möglichst klein zu halten.

Prinzipiell wird die für die Dialysebehandlung nötige Dialysierflüssigkeit unter anderem durch Lösen eines Pulvers in Wasser in/an der Dialysemaschine erzeugt, indem eine das Pulver enthaltende Kartusche von Wasser durchströmt wird. Bei dem Pulver handelt es sich im Wesentlichen um ein Natrium-Bicarbonat, wobei natürlich noch weitere Komponenten dem Wasser zugemischt werden. Auf diese Weise wird ein Flüssigkeitskonzentrat erzeugt, das später noch mit Wasser ggf. weiter verdünnt wird, um eine für die geplante Dialysebehandlung erforderliche/geeignete Konzentration zu erhalten. Da, wie vorstehend bereits angedeutet wurde, die Desinfektionsfreundlichkeit der Dialysemaschine ein wichtiges Funktionskriterium darstellt, ist es vorteilhaft, wenn die als Austauschbauteil vorgesehene und damit in der Regel außen an der Dialysemaschine angebrachte Kartusche auf möglichst einfache Weise entfernbar ist, um eine entsprechende Außenreinigung der Maschine zu ermöglichen.

### Stand der Technik

Aus der EP 1 002 550 A2 ist daher ein gattungsgemäßer Kartuschenhalter für eine Dialysemaschine bekannt, der einen unteren Haltebacken oder Arm mit einem daran ausgebildeten Ablaufanschluss und einen oberen Haltebacken oder Arm mit einem Zulaufanschluss aufweist. Zwischen diesen beiden Backen kann eine Kartusche eingesetzt werden mit einem oberen und unteren Anschlussstutzen oder Nippel, die beim Einsetzvorgang am backenseitigen Anschluss eingeführt werden müssen.

Zur Durchführung eines Spül- oder Desinfektionsvorgangs kann der obere Haltebacken in Richtung hin zum unteren Haltebacken abgesenkt werden. Bei dieser Abwärtsbewegung wird ein weiterer Zwischenbacken oder Distanzarm, der bei eingesetzter Kartusche zur Seite verschwenkt war, automatisch in Ausrichtposition mit dem unteren (und oberen) Haltebacken verschwenkt. Der Distanzarm hat dabei an seiner Ober- und Unterseite eine Nippelsimulation, welche bei weiterem Absenken der oberen Haltebacke jeweils mit den Anschlüssen an den beiden Haltebacken in Eingriff kommen und damit die Anschlüsse abdichten.

Soll die Kartusche wieder eingesetzt werden, muss der obere Haltearm aufwärts bewegt und dann die Kartuschennippel in die unteren und oberen Anschlüsse eingeführt werden. Dieser Vorgang geschieht manuell und erfordert eine Geschicklichkeit auf Seiten der Bedienerperson.

An dieser Stelle sei darauf hingewiesen, dass eine Kartuschenhalterung der einschlägigen Bauart in der Regel in Hüfthöhe an der Dialysemaschine platziert ist. Beim Einsetzten / Wechseln der Kartusche sieht die Bedienperson daher den oberen Dom / den oberen Nippel bzw. den an der oberen Haltebacke unterseitig angeordneten Anschluss nicht, sodass die richtige Stelle für das Einführen des Nippels in den oberen Anschluss regelrecht ertastet werden muss. Dies kann unabsichtlich zu Beschädigungen des oberen Kartuschennippels führen, der vergleichsweise fragil ausgebildet ist. Alternativ hierzu kann sich die Bedienperson auch soweit vorbücken, dass sie den oberen Anschluss (Anschlussöffnung) und/oder den oberen Kartuschennippel einsehen kann, wobei die permanente Wiederholung dieser Bewegung auf Dauer nicht sehr komfortabel ist.

Ferner befindet sich in/an der oberen (und unteren) Anschlussöffnung ein Schneiddorn, der eine Membran einer gattungsgemäße Kartusche für deren Öffnen durchstoßen muss. Hierfür werden entsprechend hohe Kräfte gebraucht. Eine falsche Positionierung der Kartusche bzw. des oberen Anschlussnippels/-stutzens kann beim Anstechen der Kartusche zu einer Beschädigung nicht nur des Nippels sondern auch des Schneiddorns führen, wodurch es zu Undichtigkeiten bis hin zum Funktionsverlust der Halterung kommen kann. Schließlich ist durch das nicht intuitive Einsetzen der Kartusche eine deutlich längere Handhabungszeit erforderlich.

Um daher der Notwendigkeit einer ungewünschten allgemein höheren Positionierung zu entgehen und trotzdem ein Beschädigen des oberen Kartuschennippels bzw. des oberen Schneiddorns zu vermeiden, ist es denkbar, einen Spiegel oder dergleichen optische Einrichtung an einer geeigneten Stelle anzuordnen, um den Einführvorgang zu erfassen. Es hat sich aber gezeigt, dass eine solche Einrichtung schwierig in der Verwendung ist, dass die aktuelle Nippelposition spiegelbildlich dargestellt wird und daher von der Bedienperson entsprechend umgedeutet werden muss.

Weitere Kartuschenhalter, die für Dialysemaschinen mit Haltebacken vorgesehen sind, bei denen jeweils auch das Geschick einer Bedienperson für das Einführen von Kartuschenanschlüssen in die Anschlüsse der Dialysemaschine gefordert ist, sind beispielsweise aus der DE 100 59 966 A1, der DE 199 25 297 C1, der WO 99/37342 A1 oder der WO 2010/121821 A1 bekannt.

### Kurzbeschreibung der Erfindung

In Anbetracht dieser Situation ist es die Aufgabe der vorliegenden Erfindung, eine Kartuschenhalterung dieser Gattung bereit zu stellen, welche ein sicheres Einführung des einen, visuell nicht direkt einsehbaren (oberen) Kartuschennippels in die Anschlussöffnung der oberen Haltebacke ermöglicht bzw. das Positionieren eines Kartuschenanschlusses bezüglich eines Halterungsanschlusses erleichtert. Ein Ziel ist es, Beschädigungen des Kartuschenanschlusses (Kartuschennippels) und/oder des Halterungsanschlusses während des Einsetzens der Kartusche in die Halterung zu vermeiden.

Diese Aufgabe wird durch eine Kartuschenhalterung einer Dialysemaschine mit den Merkmalen des Patentanspruchs 1 sowie durch eine Dialysemaschine mit den Merkmalen des Anspruchs 10 gelöst. Vorteilhafte Ausgestaltungen der Erfindung sind Gegenstand der Unteransprüche.

Der Kern der Erfindung besteht demnach darin, eine Kartuschenhalterung einer Dialysemaschine mit zwei im Höhenabstand zueinander angeordneten, sowie mit Anschlussöffnungen versehenen Kartuschen-Haltebacken oder Armen (zumindest eine hievon ist höhenverstell- / verschwenkbar) mit einer Kartuschen-Positionierhilfe auszustatten, die in Höhenrichtung gesehen irgendwo zwischen den beiden Haltebacken angeordnet ist. Die Positionierhilfe ist derart ausgebildet oder angepasst (mit einem Kartuschen-Eingriffsabschnitt versehen), dass sie mit der Kartusche bzw. mit zumindest einem der Kartuschenanschlüsse oder einem bestimmten Kartuschenabschnitt zumindest bei deren Einsetzvorgang in vorzugsweise formschlüssigen Eingriff kommt, derart, dass die Positionierhilfe die Kartusche in einer Position und/oder Ausrichtung (temporär) hält oder zumindest bei einer Annäherungsbewegung der beiden Haltebacken in eine Position und/oder Ausrichtung hinführt, in der sich zumindest einer der Kartuschenanschlüsse (Nippel) zu der Anschlussöffnung der oberen Haltebacke hin ausrichtet. In anderen Worten ausgedrückt, hat die Positionierhilfe einen Kartuschen-Eingriffsabschnitt, der zumindest bezüglich der einen (oberen) Haltebacke bzw. der an der einen (oberen) Haltebacke ausgebildeten Anschlussöffnung eine vorbestimmte Position hat oder während einer Verstellung der einen (oberen) Haltebacke erreicht. Diese Position ist hierbei definiert durch eine für die Halterung vorgesehene Kartusche oder durch zumindest einen an der Kartusche vorgesehenen Anschluss (-nippel), derart, dass der Kartuschenanschluss (-nippel) beim aufeinander zu Bewegen der beiden Haltebacken durch die Positionierhilfe zur zugehörigen Anschlussöffnung ausgerichtet ist oder hinbewegt wird.

Auf diese Weise übernimmt somit die Positionierhilfe gemäß der Erfindung das im Wesentlichen passgenaue Einführen insbesondere des oberen Anschlussnippels einer Kartusche in die Anschlussöffnung der oberen Haltebacke, sodass ein manuell ertastetes Einführen mit dem Risiko einer Nippelbeschädigung gemäß dem Stand der Technik entfällt.

Vorzugsweise ist die Positionierhilfe dafür angepasst, dass der Kartuschen-Eingriffsabschnitt bezüglich der oberen Haltebacke so positioniert ist, dass sich ein oberer Kartuschenanschluss (-nippel) der speziell für die jeweilige Halterung vorgesehenen Kartusche fluchtend zur oberen Anschlussöffnung ausrichtet.

Gemäß einem weiteren Aspekt der vorliegenden Erfindung besteht die Positionierhilfe aus einer Gabel mit einem (zweigezinkten) Gabelabschnitt als Kartuschen-Eingriffsabschnitt und einem Stil, dessen Länge eingestellt oder einstellbar ist, damit der Gabelabschnitt die Kartuschen-spezifische Relativposition zur Anschlussöffnung der oberen Haltebacke einnimmt oder erreichet.

Vorzugsweise ist der Gabelstil mit seinem freien Ende (gegenüber dem Gabelabschnitt) an der oberen Haltebacke montiert, um dadurch eine exakte Relativlage zwischen dem Gabelabschnitt und der oberen Anschlussöffnung unabhängig von der Montagegenauigkeit zwischen der oberen Haltebacke und der Dialysemaschine (bzw. deren Gehäuse) zu gewährleisten. In anderen Worten ausgedrückt, bilden die eine (obere) Haltebacke und die Positionierhilfe (Gabel) eine (Funktions-) Einheit oder Baugruppe, wodurch die Relativlage zwischen dem Kartuschen-Eingriffsabschnitt der Positionierhilfe und Anschlussöffnung an der zugehörigen Haltebacke unabhängig von anderen Montagefaktoren konstant bleibt.

Weiter vorzugsweise ist die eine (obere) Haltebacke höhenschwenkbar an der Dialysemaschine (bzw. deren Gehäuse) gelagert und die Positionierhilfe vorzugsweise in Form der Gabel an der verschwenkbaren Haltebacke (relativ verschwenkbar) anscharniert/angelenkt. Bei dieser Ausführungsform nimmt der Kartuschen-Eingriffsabschnitt (Gabelabschnitt) in einer aufgeschwenkten Einsetz-/Entnahmeposition eine Lage zwischen den beiden Haltebacken ein, die von einer Bedienperson gut einsehbar ist, sodass eine Kartusche exakt zwischen den Haltebacken unter in Eingriff kommen mit dem Kartuschen-Eingriffsabschnitt eingesetzt werden kann. Sobald nun die obere Haltebacke gegen die untere Haltebacke abgeschwenkt wird, verschwenkt die Positionierhilfe (Gabel) infolge des Kartuscheneingriffs als Widerlager automatisch/zwangsläufig relativ zur oberen Haltebacke um deren Anlenk-/Scharnierpunkt mit der oberen Haltebacke und führt so den oberen Kartuschenanschluss (-nippel) hin zur oberen Anschlussöffnung der oberen Haltebacke in die vorbestimmte Position.

Hierfür wäre es vorteilhaft, wenn die Positionierhilfe vorzugsweise in Form der Gabel unmittelbar mit dem einen (oberen) Kartuschenanschluss (-nippel) in (formschlüssigen) Eingriff kommt, dessen äußere Form unabhängig von der Kartuschenform selbst an die Anschlussöffnung der oberen Haltebacke nach dem Stecker-Steckdosen-Prinzip angepasst sein muss. In diesem Fall genügt eine einzige Formgebung für die Positionierhilfe entsprechend der Form der oberen Anschlussöffnung der Haltebacke sowie eine einzige vorbestimmte Position des Kartuschen-Eingriffsabschnitts in Abhängigkeit von der Position und der Form der oberen Anschlussöffnung um Kartuschen unterschiedlicher Form exakt ausrichten/positionieren zu können. D.h. in diesem Fall ist die Positionierhilfe auf die Geometrie und Position der Anschlussöffnung der oberen Haltebacke selbst (und damit intern) abgestimmt, um das gestellte Ziel der genauen Ausrichtung des Kartuschenanschlusses zu erreichen.

### Figurenbeschreibung

Die Erfindung wird nachstehend anhand eines bevorzugten Ausführungsbeispiels unter Bezugnahme auf die begleitenden Figuren näher erläutert.
Fig. 1 zeigt die prinzipielle Seitenansicht einer Kartuschenhalterung einer Dialysemaschine mit integrierter Positionierhilfe gemäß einem bevorzugten Ausführungsbeispiel der vorliegenden Erfindung;
Fig. 2 zeigt eine Seitenansicht der erfindungsgemäßen Kartuschenhalterung im Bereich ihrer oberen Haltebacke;
Fig. 3 zeigt eine Vorderansicht der erfindungsgemäßen Kartuschenhalterung im Bereich ihrer oberen Haltebacke; und
Fig. 4 zeigt eine Perspektivenansicht der erfindungsgemäßen Kartuschenhalterung im Bereich ihrer oberen Haltebacke.

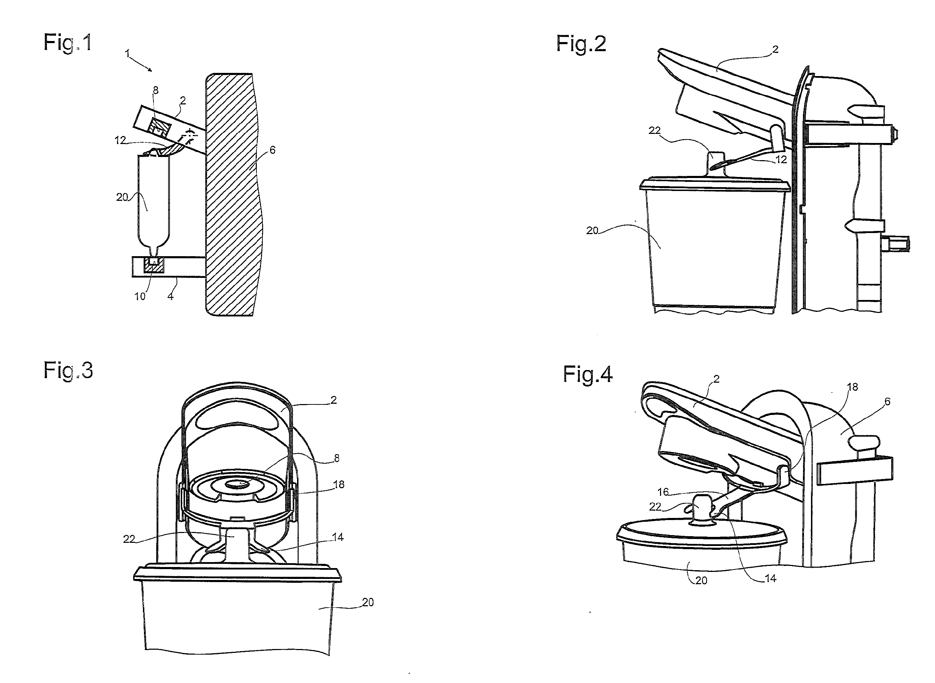

Die in der Fig. 1 schematisch dargestellte Kartuschenhalterung 1 einer Dialysemaschine 6 hat zwei höhenbeabstandete Haltebacken / - arme 2, 4, die an ihren einen Endabschnitten an der Dialysemaschine 6 (bzw. einem Gehäuseabschnitt der Dialysemaschine) montiert sind und die mit ihren jeweils anderen freien Endabschnitten von der Dialysemaschine 6 wegragen. Die untere Haltebacke 4 ist dabei vorzugsweise an der Dialysemaschine 6 (waagrecht) fixiert, wohingegen die obere Haltebacke 2 höhenverschwenkbar an der Dialysemaschine 6 angelenkt/anscharniert ist, um in eine Kartuschenentnahme- /-einsetzposition/ -stellung (entfernt von der unteren Haltebacke 4) und in eine Kartuschenanschlussposition/ -stellung (nahe zu der unteren Haltebacke 4) bewegt werden zu können.

Auf den jeweils einander zugewandten Backenseiten sindist jeweils eine Anschlussöffnung 8, 10 in den Haltebacken 2, 4 ausgeformt, die für ein mechanisches sowie strömungstechnisches in Eingriff kommen mit Kartuschenseitigen Anschlüssen ausgebildet/angepasst sind. Im Konkreten bilden die Anschlussöffnungen 8, 10 jeweils eine napfförmige sowie Dichtungs- und/oder Ventilbesetzte Einbuchtung in den Haltebacken 2, 4, in die axial zur Öffnungsrichtung sowie zentral ein hülsenförmiger Einstrechdorn (nicht detailliert dargestellt) ragt. An jeden Dorn ist eine nicht weiter gezeigte Fluidleitung angeschlossen, die innerhalb der Haltebacken 2, 4 verlegt sind und in die Dialysemaschine 6 führen.

An der oberen, schwenkbar gelagerten Haltebacke (Haltearm) 2 ist in einem Backenlängsabschnitt zwischen der oberen Anschlussöffnung 8 und dem Anlenkpunkt zur Dialysemaschine 6 eine Positionierhilfe 12, vorliegend in Form einer Gabel relativ zur oberen Haltebacke 2 höhenverschwenkbar angelenkt/anscharniert. Im Konkreten besteht die Positionierhilfe 12 gemäß der Fig. 4 aus einem freien zweigezinkten Gabelabschnitt 14, an den ein Stil 16 fixiert ist, dessen hinteres Ende an der oberen Haltebacke 2 anscharniert ist. Hierfür ist das hintere Stilende ebenfalls mit einer Schwenkgabel 18 ausgebildet/versehen, welche die obere Haltebacke 2 beidseits umgreift und mittels eines Querstifts oder mittels an der Schwenkgabel 18 (oder der Haltebacke 2) ausgeformter fluchtender Schwenkzapfen scharnierartig anmontiert ist. Vorzugsweise besteht die Gabel 12 somit aus dem freien vorderen Gabelabschnitt 14 als Kartuschen-Eingriffsabschnitt, dem ggf. längsverstellbaren Stil 16 und der hinteren Schwenkgabel 18 und ist weiter vorzugsweise stoffeinstückig aus einem Kunststoffmaterial gefertigt.

Der vordere Gabelabschnitt 14 weist in Draufsicht eine Form/Kontur auf, welche der Form/Kontur der oberen Anschlussöffnung 8 angenähert/angeglichen ist. Die Länge des Stils 16 ist ferner so gewählt/eingestellt, dass sich der vordere Gabelabschnitt 14 in einer zur oberen Haltebacke 2 hingeschwenkten Gabelposition im Wesentlichen deckungsgleich (unmittelbar) unterhalb der oberen Anschlussöffnung 8 ausrichtet/positioniert.

Die Funktion der erfindungsgemäßen Kartuschenhalterung 1 wird nachfolgend anhand der Fig. 2 bis 4 näher erläutert:
Wie aus den Fig. 2 bis 4 zu entnehmen ist, hat eine gattungsgemäße Kartusche 20 insbesondere eine Bikarbonat-Kartusche, vorgesehen zur Anwendung bei Dialysemaschinen, einen zylindrischen oder trichterförmigen langgestreckten geschlossenen Aufnahmekörper, an dessen Ober- und Unterseite jeweils ein Kartuschenanschluss 22 in Form eines Anschlussnippels ausgeformt ist. Jeder Anschlussnippel 22 ist insbesondere aus Hygienegründen vorliegend mit einer Membran (Originalitätsverschluss) abgedichtet, wobei aber auch auf eine solche Membran verzichtet werden kann, insbesondere wenn die Kartusche in einem Verpackungsbeutel luftdicht verschweißt ist.

Für das Einsetzen einer solchen Kartusche 20 in die erfindungsgemäße Halterung 1 wird zunächst die obere Haltebacke 2 in ihre Kartuschenentnahme- /-einsetzposition hochgeschwenkt, um den Höhenabstand zur unteren Haltebacke 4 zu vergrößern. Die Positionierhilfe 12, vorliegend in Form der Gabel, fällt dabei durch die Schwerkraft, bzw. optional unterstützt durch ein zusätzliches Hilfselement wie beispielsweise eine Vorspannfeder zwischen Gabel und Haltebacke in eine zur oberen Haltebacke 2 weggeschwenkte Endposition, in welches der Kartuschen-Eingriffsabschnitt 14 (Gabelabschnitt) von einer Bedienperson visuell gut erkennbar ist.

Anschließend wird eine Kartusche 20 mit dem unteren Anschluss voraus in die Halterung 1 eingesetzt, indem zuerst der untere Kartuschenanschluss /Anschlussnippel mit der Anschlussöffnung 10 der unteren Haltebacke 4 passgenau in Eingriff gebracht wird und anschließend die Kartusche 20 gegen den Eingriffsabschnitt / Gabelabschnitt 14 der Positionierhilfe 12 verschwenkt wird. Dabei greift der Gabelabschnitt 14 unter leichter Aufwärtsbewegung (gleitet an der Kartuschenoberseite geringfügig ab) um den oberen Kartuschenanschluss / Anschlussnippel 22 herum und bildet so eine Art Formschluss mit diesem, um die Kartusche 20 bzw. den oberen Anschlussnippel 22 positionsgenau in der Gabel 12 zu halten.

Wird nun die obere Haltebacke 2 gegen die untere Haltebacke 3 abwärts geschwenkt, schwenkt die Positionierhilfe / Gabel 12 aufgrund des Kartuscheneingriffs als Widerlager relativ der Haltebackenverschwenkung entgegen und führt so den oberen Kartuschenanschluss 22 zwangsläufig in die durch die Stillänge festgelegte Ausrichtposition unmittelbar unterhalb der oberen Anschlussöffnung 8. Bei einem Weiterschwenken der oberen Haltebacke 2 wird somit der Kartuschenanschluss / Anschlussnippel 22 passgenau in die obere Anschlussöffnung 8 eingeführt.

Soll bei entnommener Kartusche 20 die Halterung 1 in eine sogenannte Reinigungsposition überführt werden, in der die obere Haltebacke 2 und die untere Haltebacke 4 (vollständig) gegen die Dialysemaschine angelegt sind, wird hierfür die obere Haltebacke 2 abwärts eingeklappt, wobei sich die Positionierungshilfe / Gabel 12 automatisch an die obere Haltebacke 2 anlegt und so ein Abwärtsschwenken der oberen Haltebacke 2 bis hin zur Dialysemaschine (Gehäuse) nicht behindert.

An dieser Stelle sei darauf hingewiesen, dass in einer vereinfachten Ausführungsform die Positionierhilfe 12 fest mit der oberen Haltebacke 2 verbunden sein kann, um so eine vorbestimmte Relativposition des Kartuschen-Eingriffsabschnitts 14 der Positionierhilfe 12 zur oberen Anschlussöffnung 8 festzulegen. Weiter alternativ ist es auch möglich, die Positionierhilfe 12 nicht an der oberen Haltebacke 2 sondern unmittelbar an der Dialysemaschine 6 oder der unteren Haltebacke 4 zu fixieren, um die vorbestimmte Relativposition des Kartuschen-Eingriffsabschnitts 14 der Positionierhilfe 12 zur oberen Anschlussöffnung 8 festzulegen. In diesen alternativen Fällen kann nur die Funktion der korrekten Positionierung einer Kartusche 20 bezüglich der oberen Anschlussöffnung 8 sicher gestellt werden, wobei jedoch beispielsweise für ein Überführen der oberen Haltebacke 2 in die Reinigungsstellung die Positionierhilfe 12 entfernt werden müsste.

Die Positionierhilfe 12 ist aus einem hochfesten Kunststoff (z.B. PBT-ASA GF20) und kann bei Bedarf ohne Werkzeug demontiert werden. So ist eine leichte Reinigung möglich und es ist eine eventuell nicht kompatible Kartusche durch Austausch der Gabel dennoch verwendbar. Der Kunststoff ist in der Regel beständig gegen die meisten Desinfektionsmittel. Die Positionierhilfe 12 kann aber auch aus einem Metall gefertigt sein. Sie hat dann ein gegenüber dem Kunststoff höheres Gewicht und erreicht damit die Einsetzposition schwerkraftbedingt auch ohne zusätzliches Vorspannmittel sicher.

Zusammenfassend wird vorliegend eine Kartuschenhalterung 1 für eine (bzw. einer) Dialysemaschine 6 offenbart zum Befestigen und Anschließen einer Kartusche 20, insbesondere einer Bikarbonat-Kartusche zwischen zwei relativ zueinander bewegbaren Halteorganen (Armen) 2, 4, die zumindest zwischen einer Einsetzstellung, in der die Kartusche 20 eingesetzt und/oder herausgenommen werden kann und einer Betriebsstellung bewegbar sind, in der die Kartusche 20 von den beiden Halteorganen 2, 4 gehalten wird und mit einem Zu- und Ablauf 8, 10 verbunden oder verbindbar ist. Ferner ist eine Positionierhilfe 12 vorgesehen, die in der Einsetzstellung der Halteorgane 2, 4 einen Anschlussstutzen oder Nippel 22 der Kartusche 20 positionsgenau aufnehmen kann und den Anschlussstutzen 22 zu einem in einem entsprechenden Halteorgan 2 vorgesehenen Anschluss 8 führen kann, wenn die Halteorgane 2, 4 in die Betriebsstellung gebracht werden.

### Bezugszeichenliste

- 1: Kartuschenhalterung
- 2, 4: Haltebacken
- 6: Dialysemaschine
- 8, 10: Anschlussöffnungen
- 12: Positionshilfe
- 14: Gabelabschnitt
- 16: Stil
- 18: Schwenkgabel
- 20: Kartusche
- 22: Kartuschennippel

## Patentansprüche

1. Kartuschenhalterung einer Dialysemaschine mit zwei im Höhenabstand zueinander angeordneten Haltebacken (2, 4), von denen zumindest eine Haltebacke (2) in Höhenrichtung relativ zur anderen Haltebacke (4) bewegbar, oder verschwenkbar ist und an deren einander zugewandten Seiten jeweils eine Anschlussöffnung (8, 10) zur Aufnahme jeweils eines Anschlusses (22) einer darin eingesetzten oder einzusetzenden Kartusche (20) vorgesehen ist, und mit einer zwischen den beiden Haltebacken (2, 4) angeordneten Kartuschen-Positionierhilfe (12),
**dadurch gekennzeichnet,**
**dass** die Positionierhilfe (12) für ein unmittelbares in Eingriff kommen mit zumindest einem der Kartuschenanschlüsse (22) der Kartusche (20) zumindest bei deren Einsetzvorgang ausgebildet ist, derart, dass die Positionierhilfe (12) den zumindest einen der Kartuschenanschlüsse (22) in einer Position und/oder Ausrichtung hält oder zumindest bei einer Annäherungsbewegung der beiden Haltebacken (2, 4) in eine Position und/oder Ausrichtung hinführt, in der sich zumindest einer der Kartuschenanschlüsse (22) zu der Anschlussöffnung (8) der oberen Haltebacke (2) hin ausrichtet.

2. Kartuschenhalterung nach Anspruch 1, **dadurch gekennzeichnet, dass** die Positionierhilfe (12) einen Kartuschen-Eingriffsabschnitt (14) hat, der bezüglich der oberen Haltebacke (2) so positioniert ist, dass er sich bei eingesetzter Kartusche (20) im Wesentlichen deckungsgleich unterhalb der Anschlussöffnung (8) der oberen Haltebacke (2) anordnet, sodass sich ein oberer Kartuschenanschluss (22) im Wesentlichen fluchtend zur Anschlussöffnung (8) der oberen Haltebacke (2) ausrichtet.

3. Kartuschenhalterung nach Anspruch 2, **dadurch gekennzeichnet, dass** die Positionierhilfe (12) aus einer Gabel mit einem zweigezinkten Gabelabschnitt (14) als Kartuschen-Eingriffsabschnitt und einem Stil (16) besteht, dessen Länge eingestellt oder einstellbar ist, damit der Gabelabschnitt (14) die Relativposition zur Anschlussöffnung (8) der oberen Haltebacke (2) einnimmt oder erreichet.

4. Kartuschenhalterung nach Anspruch 3, **dadurch gekennzeichnet, dass** der Gabelstil (16) mit seinem einen dem Gabelabschnitt (14) gegenüber liegenden Längsende an der oberen Haltebacke (2) montiert ist, um mit dieser eine Funktionseinheit zu bilden.

5. Kartuschenhalterung nach Anspruch 3, **dadurch gekennzeichnet, dass** die obere Haltebacke (2) höhenschwenkbar an der Dialysemaschine (6) gelagert ist und die Positionierhilfe (12) in Form der Gabel an der verschwenkbaren Haltebacke (2) relativ verschwenkbar anscharniert ist.

6. Kartuschenhalterung nach Anspruch 5, **dadurch gekennzeichnet, dass** der Kartuschen-Eingriffsabschnitt (14) der Positionierhilfe (12) auf die Geometrie und Position der Anschlussöffnung (8) der oberen Haltebacke (2) abgestimmt ist, derart, dass sich der Kartuschen-Eingriffsabschnitt (14) in einer Kartuschen-Betriebsstellung im Wesentlichen überdeckend unterhalb der oberen Anschlussöffnung (8) anordnet.

7. Dialysemaschine mit einer Kartuschenhalterung gemäß einem der vorstehenden Ansprüche 1 bis 6.

8. Dialysemaschine nach Anspruch 7, **dadurch gekennzeichnet, dass** die untere Haltebacke (4) an einem Gehäuseabschnitt oder Rahmenteil der Dialysemaschine (6) fixiert oder zwischen einer Betriebsstellung und einer Spül-/Reinigungsstellung hin- und her klappbar gehalten ist und die obere Haltebacke (2) an dem Gehäuseabschnitt oder Rahmenteil der Dialysemaschine (6) höhenverschwenkbar angelenkt ist, um zumindest aus der Betriebsstellung in eine Kartuschen-Entnahmestellung verschwenkt zu werden.

9. Dialysemaschine nach Anspruch 7 oder 8, **dadurch gekennzeichnet, dass** zumindest die obere Haltebacke (2) in eine Reinigungsstellung umklappbar ist, in der sie sich unter Relativverschwenken der Positionierhilfe (12) in Richtung hin zur oberen Haltebacke (2) an die Dialysemaschine (6) anlegt.

10. Dialysemaschine nach Anspruch 9, **dadurch gekennzeichnet, dass** auch die untere Haltebacke (4) in eine Reinigungsstellung umklappbar ist, in der sie sich unter Relativverschwenken der Positionierhilfe (12) in Richtung hin zur oberen Haltebacke (2) an die Dialysemaschine (6) anlegt.

## Claims

1. A cartridge holder of a dialyzer comprising two vertically spaced retaining jaws (2, 4), at least one retaining jaw (2) being vertically movable or pivotable relative to the other retaining jaw (4) and each of said retaining jaws having, on the sides facing one another, formed therein a connection opening (8, 10) for receiving therein a respective connection (22) of a cartridge (20) which is inserted or which is to be inserted therein, and comprising a cartridge positioning aid (12) arranged between the two retaining jaws (2, 4)
**characterized in**
**that** the positioning aid (12) is configured for direct engagement with at least one of the cartridge connections (22) of the cartridge (20), at least during insertion of the latter, such that the positioning aid (12) holds the at least one cartridge connection (22) at a position and/or orientation or at least leads the cartridge (20) during a movement, with which the two retaining jaws (2, 4) approach one another, to a position and/or orientation in which at least one of the cartridge connections (22) will orient itself towards the connection opening (8) of the upper retaining jaw (2).

2. The cartridge holder according to claim 1, **characterized in that** the positioning aid (12) has a cartridge engagement portion (14) which is positioned relative to the upper retaining jaw (2) such that, in the installed condition of the cartridge (20), it will be arranged in a substantially congruent manner below the connection opening (8) of the upper retaining jaw (2), so that an upper cartridge connection (22) will orient itself such that it is substantially in alignment with the connection opening (8) of the upper retaining jaw (2).

3. The cartridge holder according to claim 2, **characterized in that** the positioning aid (12) consists of a fork comprising a preferably two-prong fork portion (14) as a cartridge engagement portion and a shaft (16) whose length is adjusted or adjustable so that the fork portion (14) occupies or arrives at the position relative to the connection opening (8) of the upper retaining jaw (2).

4. The cartridge holder according to claim 3, **characterized in that** the fork shaft (16) has its longitudinal end, which is located opposite the fork portion (14), mounted on the upper retaining jaw (2) so as to define a functional unit therewith.

5. The cartridge holder according to claim 3, **characterized in that** the upper retaining jaw (2) is supported on the dialyzer (6) in a vertically pivotable manner, and that the positioning aid (12) in the form of the fork is articulated on the pivotable retaining jaw (2) such that it is pivotable relative thereto.

6. The cartridge holder according to claim 5, **characterized in that** the cartridge engagement portion (14) of the positioning aid (12) is adapted to the geometry and position of the connection opening (8) of the upper retaining jaw (2) such that, in a cartridge operating position, the cartridge engagement portion (14) will be arranged below the upper connection opening (8) in a substantially congruent fashion.

7. A dialyzer comprising a cartridge holder according to one of the preceding claims 1 to 6.

8. The dialyzer according to claim 7, **characterized in that** the lower retaining jaw (4) is fixed to a housing area or frame section of the dialyzer (6) or supported such that it can be pivoted between an operating position and a flushing/cleaning position, and that the upper retaining jaw (2) is articulated on the housing area or frame section of the dialyzer (6) such that it is vertically pivotable for pivoting at least from the operating position to a cartridge removal position.

9. The dialyzer according to claim 7 or 8, **characterized in that** at least the upper retaining jaw (2) can be folded into a cleaning position, at which it assumes a position of full contact with the dialyzer (6) with relative pivoting of the positioning aid (12) towards the upper retaining jaw (2).

10. The dialyzer according to claim 9, **characterized in that** also the lower retaining jaw (4) can be folded into a cleaning position, at which it assumes a position of full contact with the dialyzer (6) with relative pivoting of the positioning aid (12) towards the upper retaining jaw (2).

## Revendications

1. Support de cartouche d'un appareil de dialyse, comportant deux mâchoires de retenue (2, 4) situées à une distance verticale l'une de l'autre, parmi lesquelles au moins une mâchoire de retenue (2) est mobile verticalement par rapport à l'autre mâchoire de retenue (4) ou est apte à pivoter, et sur leurs faces orientées l'une vers l'autre est prévue respectivement une ouverture de raccordement (8, 10) pour recevoir respectivement un raccord (22) d'une cartouche (20) insérée ou à insérer, et comportant une aide au positionnement de la cartouche (12) disposé entre les deux mâchoires de retenue (2, 4),
**caractérisé en ce que**
l'aide au positionnement (12) est réalisé pour une entrée en prise directe avec au moins un des raccords (22) de la cartouche (20) au moins pendant le processus de mise en place de celle-ci, de telle sorte que l'aide au positionnement (12) maintient ledit au moins un des raccords (22) de la cartouche dans une position et/ou dans une orientation ou, du moins pendant un mouvement de rapprochement des deux mâchoires de retenue (2, 4), le mène dans une position et/ou orientation, dans laquelle au moins un des raccords (22) de la cartouche est orienté vers l'ouverture de raccordement (8) de la mâchoire de retenue (2) supérieure.

2. Support de cartouche selon la revendication 1, **caractérisé en ce que** l'aide au positionnement (12) possède un tronçon d'engagement (14) dans la cartouche, qui est positionné par rapport à la mâchoire de retenue (2) supérieure de telle sorte que, lorsque la cartouche (20) est en place, ledit tronçon d'engagement est disposé sous l'ouverture de raccordement (8) de la mâchoire de retenue (2) supérieure de manière à coïncider sensiblement avec ladite ouverture, de telle sorte qu'un raccord de cartouche (22) supérieur est aligné sensiblement avec l'ouverture de raccordement (8) de la mâchoire de retenue (2) supérieure.

3. Support de cartouche selon la revendication 2, **caractérisé en ce que** l'aide au positionnement (12) est constituée d'une fourche avec une partie (14) à deux dents, formant le tronçon d'engagement dans la cartouche, et d'une tige (16), dont la longueur est réglée ou réglable pour que la partie de fourche (14) prenne ou atteigne la position relative par rapport à l'ouverture de raccordement (8) de la mâchoire de retenue (2) supérieure.

4. Support de cartouche selon la revendication 3, **caractérisé en ce que** la tige (16) de la fourche, avec son extrémité longitudinale opposée à la partie de fourche (14), est montée sur la mâchoire de retenue (2) supérieure afin de former avec celle-ci une unité fonctionnelle.

5. Support de cartouche selon la revendication 3, **caractérisé en ce que** la mâchoire de retenue (2) supérieure est montée de manière à pouvoir pivoter verticalement sur l'appareil de dialyse (6) et l'aide au positionnement (12) en forme de fourche est attachée par des charnières à la mâchoire de retenue (2) de manière à pouvoir pivoter par rapport à cette dernière.

6. Support de cartouche selon la revendication 5, **caractérisé en ce que** le tronçon d'engagement dans la cartouche (14) de l'aide au positionnement (12) est ajusté à la géométrie et à la position de l'ouverture de raccordement (8) de la mâchoire de retenue (2) supérieure, de telle sorte que le tronçon d'engagement (14), dans une position de service de la cartouche, est disposé sous l'ouverture de raccordement (8) supérieure en masquant sensiblement celle-ci.

7. Appareil de dialyse, comportant un support de cartouche selon l'une des revendications 1 à 6.

8. Appareil de dialyse selon la revendication 7, **caractérisé en ce que** la mâchoire de retenue (4) inférieure est fixée à une partie du carter ou partie du châssis de l'appareil de dialyse (6) ou est maintenue de manière à pouvoir basculer en va-et-vient entre une position de service et une position de lavage/nettoyage, et la mâchoire de retenue (2) supérieure est articulée de manière pivotante verticalement contre la partie du carter ou partie du châssis de l'appareil de dialyse (6), afin de pouvoir la faire pivoter au moins hors de la position de service dans une position d'extraction de la cartouche.

9. Appareil de dialyse selon la revendication 7 ou 8, **caractérisé en ce qu'**au moins la mâchoire de retenue (2) supérieure peut basculer dans une position de nettoyage, dans laquelle elle est en appui contre l'appareil de dialyse (6) moyennant un pivotement relatif de l'aide au positionnement (12) vers la mâchoire de retenue (2) supérieure. _

10. Appareil de dialyse selon la revendication 9, **caractérisé en ce que** la mâchoire de retenue (4) inférieure peut aussi basculer dans une position de nettoyage, dans laquelle elle est en appui contre l'appareil de dialyse (6) moyennant le pivotement relatif de l'aide au positionnement (12) vers la mâchoire de retenue (2) supérieure.
